# EUROPEAN PATENT APPLICATION

(11) **EP 4 723 054 A1**
(43) Date of publication of application: **08.04.2026**
(21) Application number: 24815473.4
(22) Date of filing: 28.05.2024
(51) Int. Cl.: G06V 10/774, A61B 5/00, G06T 7/00, G16H 30/20

(54) **PROGRAM, GENERATION METHOD, AND SYSTEM**

(30) Priority: 31.05.2023 JP 2023090399
(71) Applicant: Denka Company Limited, Tokyo 103-8338 (JP)
(72) Inventor: KOBUNA, Hiroyuki, Tokyo 103-8338 (JP); FUKUDA, Koichi, Tokyo 103-8338 (JP); ITAKURA, Satoshi, Tokyo 103-8338 (JP); OKUDA, Shujiro, Niigata-shi, Niigata 950-0917 (JP)
(74) Representative: Müller-Boré & Partner Patentanwälte PartG mbB
(86) International application number: PCT/JP2024/019506
(87) International publication number: WO 2024/247990

(57) **Abstract**

A program, a generation method, and a system that provide a novel and highly convenient training data generation technology are provided. A program for generating training data from a medical image is provided. The program causes a computer to execute a generation process, a plurality of pieces of information are associated with a region image, which is an image of at least one region in the medical image, and the generation process causes the computer, by a manual operation received via an operation input unit or by an automatic process of the computer, to perform a label association with the region image based on the plurality of pieces of information, thereby generating the training data from the medical image.

## Description

### Technical Field

The present invention relates to a program, a generation method, and a system for generating training data for a machine learning algorithm.

### Background Art

A data generation device of Patent Literature 1 generates training data for a machine learning algorithm.

### Citation List

### Patent Literature

[Patent Literature 1] International Publication No. 2021/206053

### Summary of Invention

### Technical Problem

For the generation of training data for medical images, there is a situation in which a person with expert knowledge manually labels a large number of images one by one. Such a situation is due to the fact that a high degree of expertise, accuracy, and the like are required for the judgment of the medical images. However, a work burden of manually labeling the large number of images has become a problem. A more convenient training data generation technology has been desired.

The present invention provides a program, a generation method, and a system that provide a novel and highly convenient training data generation technology.

### Solution to Problem

According to the present disclosure, at least the following inventions are provided.
[1] A program for generating training data from a medical image, wherein the program causes a computer to execute a generation process, a plurality of pieces of information are associated with a region image, which is an image of at least one region in the medical image, and the generation process causes the computer, by a manual operation received via an operation input unit or by an automatic process of the computer, to perform a label association with the region image based on the plurality of pieces of information, thereby generating the training data from the medical image.
[2] The program according to [1], wherein the plurality of pieces of information include a first information and a second information, the first information is a first label, the second information is a second label, and the program causes the computer to execute a process of acquiring the first label and the second label associated with one of the region images by a first annotation and a second annotation.
[3] The program according to [1] or [2], wherein each of the plurality of pieces of information is a label, and the generation process causes the computer, by a manual operation received via the operation input unit or by an automatic process of the computer, to determine a determined label of the one region image from a plurality of labels associated with the one region image, and to associate a training data image generated from the medical image with the determined label, thereby generating the training data.
[4] The program according to [1], wherein the plurality of pieces of information include a first information and a second information, the region image includes a first region image and a second region image, and the generation process causes the computer to associate a label determined for the first region image based on the first information with the second region image that has been selected based on the second information, thereby generating the training data.
[5] The program according to [4], wherein the program causes the computer to execute a process of acquiring an input data including a plurality of the region images, a plurality of the first region images included in the plurality of the region images are generated from each of a first medical image and a second medical image, the first and second medical images are associated with the first information, the first medical image is an image in which the first information satisfies a predetermined criterion, the second medical image is an image in which the first information does not satisfy the criterion, the program causes the computer to execute a discrimination process of causing a trained discrimination model to perform a machine inference for classifying a plurality of the second region images included in the plurality of the region images of the input data, a classification result of the region image by the machine inference is the second information, and the generation process causes the computer to associate a label indicating that the first information satisfies the criterion with the second region image that is included in the first region image generated from the first medical image and classified into a first classification group as the classification result, and to associate a label indicating that the first information does not satisfy the criterion with the second region image that is included in the first region image generated from the second medical image and classified into the first classification group as the classification result, thereby generating the training data.
[6] The program according to [4] or [5], wherein the medical image includes a pathology image.
[7] The program according to [6], wherein the first information includes at least one of a gene mutation type, a TMB-H (Tumor Mutational Burden-High) type, a result of a biochemical analysis, or a patient's age, sex, medical history, a result of an immunohistochemical analysis, a lifestyle habit, or a prognostic prediction.
[8] The program according to any one of [1] to [7], wherein at least one of the plurality of pieces of information comprises an index value or is an index value, and the index value represents a certainty of a content of the information.
[9] The program according to [8], citing [2] or [3], wherein a label associated with the region image comprises an index value or is an index value, and the index value represents how certain a classification result of the label is.
[10] The program according to [8], citing [5], wherein the trained discrimination model outputs, by the machine inference, a probability value representing a certainty of a classification result of the region image, and the probability value is used as the index value.
[11] A generation method for generating training data from a medical image, the method causing a computer to execute a generation step, a plurality of pieces of information are associated with a region image, which is an image of at least one region in the medical image, and the generation step causes the computer, by a manual operation received via an operation input unit or by an automatic process of the computer, to perform a label association with the region image based on the plurality of pieces of information, thereby generating the training data from the medical image.
[12] A system for generating training data from a medical image, comprising a generation unit, a plurality of pieces of information are associated with a region image, which is an image of at least one region in the medical image, and the generation unit, by a manual operation received via an operation input unit or by an automatic process of a computer, performs a label association with the region image based on the plurality of pieces of information, thereby generating the training data from the medical image.

According to the present invention, a novel technology is provided for performing label association with a region image based on a plurality of pieces of information associated with the region image. This makes it possible to generate the training data with high convenience.

### Brief Description of Drawings

FIG. 1 is a diagram illustrating an example of a system 1 (a server 1), each user terminal 3, and a diagnosis device 4 according to an embodiment.
FIG. 2A is a block diagram illustrating an example of a hardware configuration of the server 1.
FIG. 2B is a block diagram illustrating an example of a hardware configuration of the user terminal 3.
FIG. 3A is a block diagram showing a functional configuration of the server 1 of a first embodiment.
FIG. 3B is a functional block diagram of a generation unit 23 of the first embodiment.
FIG. 4 is a first example of a processing flow (a grid method, a manual determination process) representing a control operation of the first embodiment.
FIG. 5 is a second example of a processing flow (a grid method, an automatic determination process) representing the control operation of the first embodiment.
FIG. 6 is a third example of a processing flow (a designated frame method, a manual determination process) representing the control operation of the first embodiment.
FIG. 7A illustrates a state in which a grid display is turned on in a selection screen 27 of FIG. 6 (the designated frame method).
FIG. 7B is an example of a result of performing a region image conversion according to the grid of FIG. 7A.
FIG. 8 is a fourth example of a processing flow (a designated frame method, an automatic determination process) representing the control operation of the first embodiment.
FIG. 9A illustrates a case where there are three designated frames in FIG. 7A.
FIG. 9B is an example of a result of performing a region image conversion according to the grid of FIG. 9A.
FIG. 10A is an example of an image in which a label comprises an index value.
FIG. 10B is an example of an image in which a label is an index value.
FIG. 11A is a block diagram showing a functional configuration of the server 1 of a second embodiment.
FIG. 11B is a functional block diagram of the generation unit 23 of the second embodiment.
FIG. 12 is a first example of a processing flow representing a control operation of the second embodiment.
FIG. 13 is a second example of a processing flow (a process using an index value) representing the control operation of the second embodiment.

### Description of Embodiments

Hereinafter, some embodiments of the present invention will be described with reference to the drawings. Various features shown in the embodiments described below can be combined with each other. In addition, an invention is independently established for each feature.

### <1. Term, etc.>

### (1-1. Annotation)

In the field of machine learning, the term annotation, depending on the context, refers to an act of annotating by labeling an image, or refers to a group of assigned labels. The annotation as the act of annotating means "an act of associating annotation information as correct answer data with target information (electronic information) for machine learning". Various data such as images, videos, and audio can be targets of the annotation, and for example, an image annotation includes an object detection, a region image extraction, and an image classification. The image annotation associates a label with an annotation target. The annotation target may be each image (an entire image) or a region image (an area, including the entire image) in the image. The label is also referred to as a tag or metadata. The label represents a classification content (a classification result) and becomes the correct answer data during a supervised learning. In the embodiments, "the annotation", unless a different definition is specified or an obvious contradiction arises in the context, shall mean "associating a label with a region image, which is an image of this region, when at least one region is designated in an image".

### (1-2. Image to be Annotated)

An image to be annotated in the embodiments shall include at least a "medical image". The medical image includes "an image that visualizes a structure or a function inside a human body as an image, mainly for a diagnosis and a treatment of a disease". The medical image may specifically include a simple X-ray photography, a computed tomography (CT), a magnetic resonance imaging (MRI), an ultrasonic tomographic image (US), a nuclear medicine examination, an angiography, and the like. The medical image according to the present disclosure may further include an "endoscopic image" and a "pathology image" in addition to the various images described above. The endoscopic image may include, for example, images of an esophagus, a stomach, an intestine, and other digestive systems. The pathology image may specifically be "a (microscopic) image obtained by enlarging and photographing a specimen, which is a tissue collected by an endoscopy, a surgery, or the like, that has been (1) fixed to prevent deterioration, (2) stained to facilitate observation, and (3) thinly sectioned to about several microns".

The present invention is similarly applicable to any medical image, and any label classification type and classification content can be set. A label classification type is, for example, information regarding a type of pathology. As an example, the type of pathology may be either "tumor" or "hypermutation". For example, when each of the medical images is an image in which the presence of a tumor is suspected, "tumor" may be set as the type of pathology, and in that case, a classification content of a label can be "tumor" or "non-tumor". When each of a plurality of the medical images is an image in which the presence of a hypermutation is suspected, "hypermutation" may be set as the type of pathology, and in that case, the classification content of the label can be "hypermutation" or "non-hypermutation". In a first embodiment and a second embodiment below, a pathology image is adopted as an example of the medical image, the label classification type is, as an example, a classification of whether it is a tumor, and the classification content of the label is assumed to be "tumor" or "non-tumor".

In the embodiments, a pathology image is adopted as an example of the medical image, the label classification type is a classification of whether it is a tumor, and the classification content of the label is "tumor" or "non-tumor".

### (1-3. Region and Region Image)

There are various methods for designating a region (an area) in a medical image. The embodiments adopt a grid method in some examples and a designated frame method in other examples.

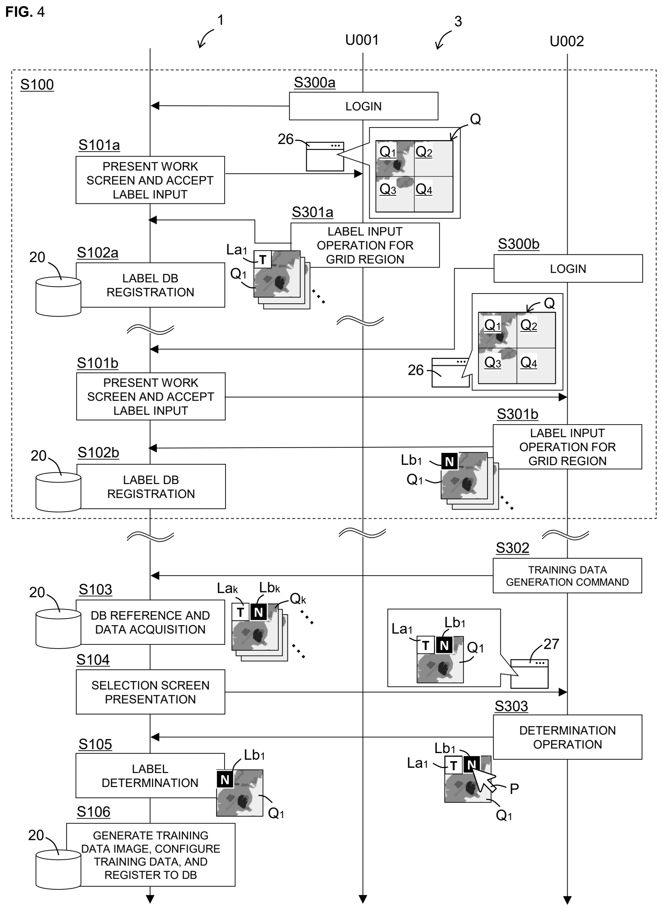

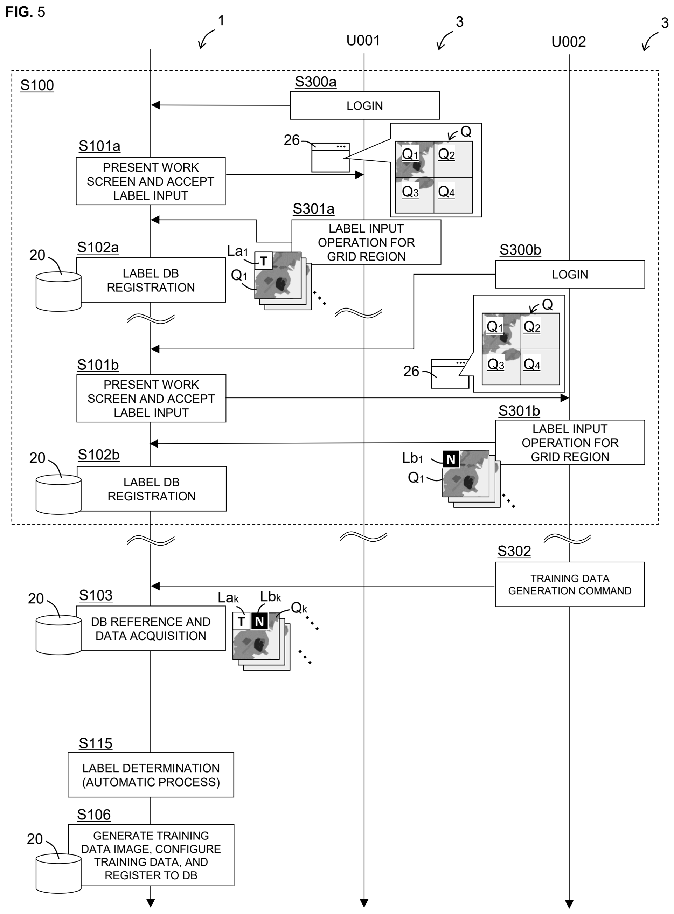

The "grid method" designates a region at a desired position and range in an image by a grid (that is, a lattice-like boundary line). When the grid has a plurality of cells, one of the cells of the grid can define one region. The grid may have uniform intervals, each region may be designated as a rectangle of a uniform size, and such a region may be referred to as a "tile". A grid interval is either defined or can be arbitrarily designated by a user. Note that the "grid" mentioned here has a broad meaning and is not limited to a rectangular lattice, and a lattice in which arbitrary polygons that can be tiled on a plane are periodically arranged can be adopted. The grid may be, for example, a honeycomb grid or the like. The "honeycomb grid" mentioned here has a broad meaning, and may be, for example, a regular hexagonal honeycomb, but may also be, for example, a honeycomb structure of regular triangles or parallelograms, or a honeycomb structure of parallelohexagons (a hexagon in which three pairs of opposite sides are parallel but the side lengths differ between each pair). In the embodiments, a grid-method annotation is adopted in the examples of FIG. 4 and FIG. 5.

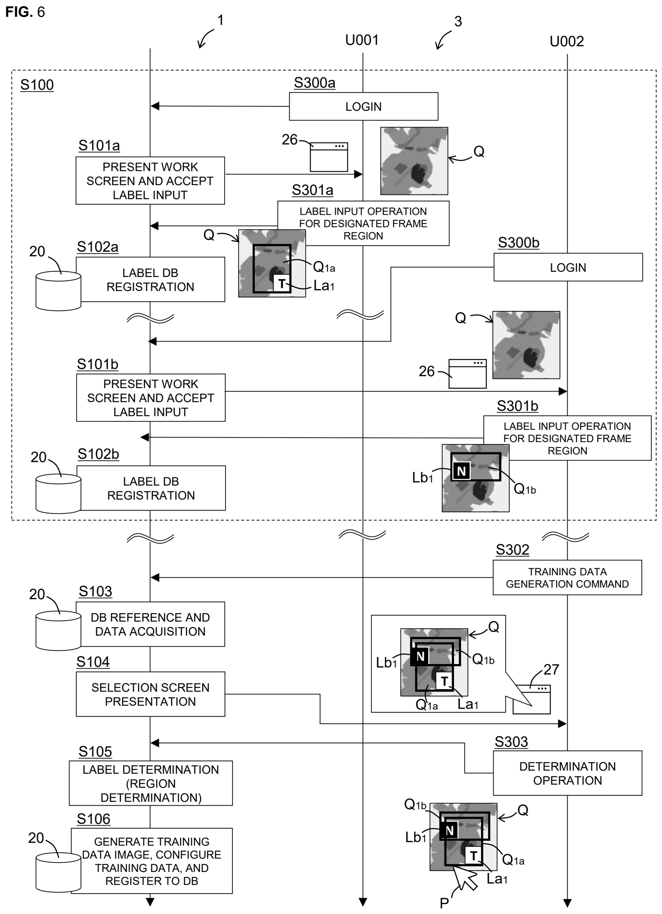

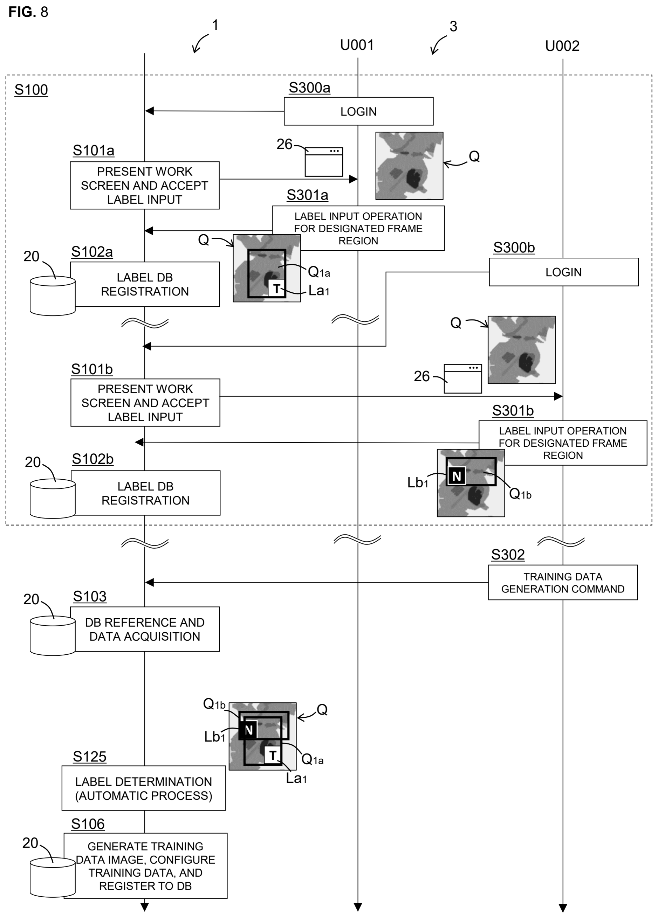

The designated frame method designates a region at a desired position and range in an image by a designated frame arbitrarily designated by a user. The designated frame is sometimes referred to as a bounding box. The designated frame can be set by the user to an arbitrary size and an arbitrary shape. The designated frame may be, for example, a rectangle, but is not limited thereto, and may be an arbitrary polygon or a closed curve. In the embodiments, a designated-frame-method annotation is adopted in the examples of FIG. 6 and FIG. 8.

In the following description, for convenience, a region designated by a grid may be referred to as a "grid region" to facilitate understanding. Also, for convenience, a region designated by a designated frame may be referred to as a "designated frame region" to facilitate understanding. On the other hand, when simply described as a "region", it may be intended to include both the grid region and the designated frame region, except when it is clear from the context that it refers to one of the grid region and the designated frame region.

When a region is designated in an image, an image corresponding to the region is also referred to as a "region image". When the region image is an image designated in a uniform rectangular size by the grid method, this region image may be referred to as a tile image. A size (a number of pixels in a vertical direction and a horizontal direction) of the region and the region image may be the same size as the training data that can be input to a learning model. When a plurality of designated frames are set in an image, if two designated frames completely match, the designated frame regions match each other, but if each designated frame is different, the designated frame regions are also different. When there is an overlap between the designated frames, an overlapping region where a plurality of the designated frame regions overlap occurs.

The program, method, and system of the embodiments can adopt an annotation of one or both of the grid method and the designated frame method. When both are adopted, which method to use in a current annotation may be set by a user's setting operation and/or by an automatic computer process.

### (1-4. Training Data)

Usually, an annotation is performed to configure a training data set. When annotating a medical image, the training data set is composed of a "training data image" obtained by cropping the medical image and a label associated with the training data image. A size (a number of pixels in a vertical direction and a horizontal direction) of the training data image is set to a size that can be input to the learning model. The "training data image" may be one obtained by cropping the medical image along a grid, or may be one obtained by cropping a part of the medical image with a designated frame (a bounding frame). The training data image may be one obtained by cropping the medical image according to the region image at the time of annotation, that is, the region image at the time of annotation and the training data image may match. However, the present invention is not limited to this, and the training data image may be different from the region image at the time of annotation. As an example, when a medical image is annotated with a designated frame, the training data image may be generated by cropping this with a grid (for example, a rectangular grid with uniform intervals). As another example, when an annotation is made with a honeycomb grid or the like, the training data image may be generated by cropping this with another grid (for example, a rectangular grid with uniform intervals).

It should be noted that cropping of a medical image is not necessary at a stage during an annotation work or at a stage before a training data generation process. For example, each region image of a medical image may be displayed on a work screen 26 and a selection screen 27 described later, but cropping of the medical image is not necessary at this display stage. Cropping of the medical image may be performed at a stage where the annotation is completed and then an instruction to create a training data set is issued.

### <2. First Embodiment>

### (2-1. Overview of System 1 and Diagnosis Device 4)

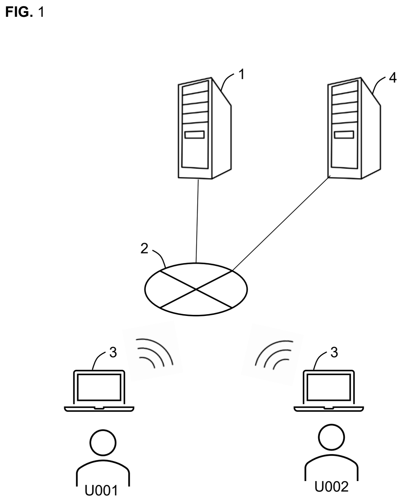

As shown in FIG. 1, a system 1, a user terminal 3, and a diagnosis device 4 of a first embodiment can communicate with each other via a wireless or wired communication network 2. The system 1 of FIG. 1 can generate training data from a medical image. The system 1 is a computer, and specifically, is a server. The system 1 is provided by a server executing a program. For convenience, the system 1 may be referred to as a server 1. Users U001 and U002 who use the system 1 are, for example, pathologists.

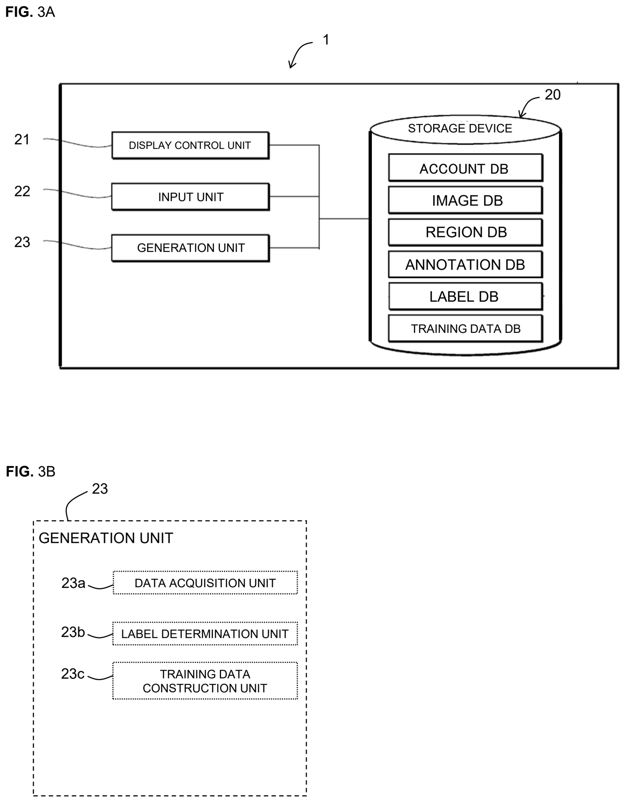

The system 1 includes a generation unit 23 (see FIG. 3A) having a function of generating training data. The generation unit 23 can generate the training data using an input image (specifically, a medical image). The training data can be used as teacher data for training a learning model provided in the diagnosis device 4. The diagnosis device 4 uses a trained model that has learned using the generated training data to determine whether a pathological abnormality exists in a medical image.

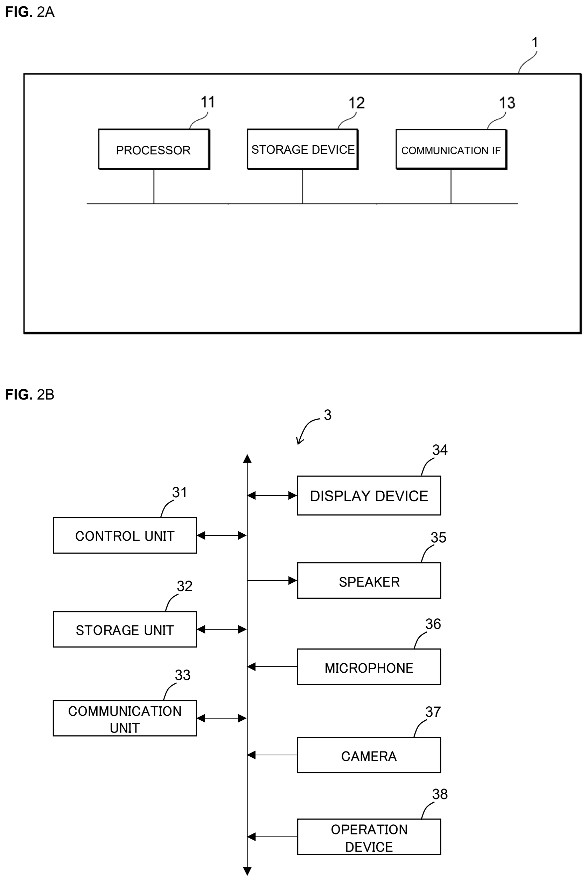

The system 1 can display a medical image (a pathology image in the embodiment) stored in a storage unit 20 and a grid (boundary lines of a plurality of regions) superimposed on a screen. An example of the screen is a screen on a display device 34 (see FIG. 2B) of the user terminal 3. The system 1 accepts an annotation operation for a region image by a user. That is, the system 1 accepts an input of a label from the users U001 and U002 for each one of the region images displayed on the screen. As a GUI for a label input operation, the system 1 generates a work screen 26 in FIG. 4, which will be described later, and causes the user terminal 3 to display it.

The system 1 may generate the training data from one pathology image, or may generate the training data from a plurality of pathology images. When generating the training data from a plurality of the pathology images, the system 1 repeats a process of dividing a pathology image into a plurality of region images and accepting an input of a label for each pathology image. When the user has completed assigning labels to all the pathology images, the training data is generated. For example, the system 1 generates a training data image by cropping the medical image according to the grid, and generates the training data by associating image data of this training data image with the label assigned to each region image. The generated training data is sent to the diagnosis device 4.

The diagnosis device 4 trains (learns) the learning model using the training data sent from the system 1. A specific configuration of the learning model is not limited, and may be, for example, a neural network that can be given a predetermined ability by machine learning. The diagnosis device 4 inputs a pathology image to be diagnosed into the trained model generated by machine learning, and based on an output result from the trained model, determines whether a pathological abnormality exists in the pathology image.

The system 1 can provide a selection screen 27 as an assist function to assist an annotation. The assist function will be described in detail later.

### (2-2. Hardware Configuration of System 1)

FIG. 2A is a diagram illustrating an example of a hardware configuration of the system 1. The system 1 includes a processor 11 such as a CPU (Central Processing Unit) or a GPU (Graphical Processing Unit), a memory, a storage device 12 such as an HDD (Hard Disk Drive) and/or an SSD (Solid State Drive), and a communication IF (Interface) 13 for performing wired or wireless communication. The system 1 accepts a user input operation from the user terminal 3 via the communication IF 13, and causes the display device 34 (see FIG. 2B) of the user terminal 3 to display various screens.

### (1-3. Hardware Configuration of User Terminal 3)

As shown in FIG. 2B, the user terminal 3 includes, as an example, a control unit 31 including a processor and the like, a storage unit 32 including a non-volatile memory and the like, a communication unit 33 for performing wireless communication and/or wired communication, a display device 34, a speaker 35, a microphone 36, a camera 37, and an operation device 38. The operation device 38 is an example of an operation input unit, and is any input device such as a keyboard or a mouse. A specific configuration of the user terminal 3 is not limited, and may be various mobile terminals, smartphones, tablet terminals, or notebook PCs. When the user terminal 3 has a touch panel display, the display device 34 and the operation device 38 may be integrated into the touch panel display. The operation device 38 and the microphone 36 serve as an input interface (or an input means) for a user to perform various input operations on the user terminal 3.

### (2-4. Functional Block Configuration of System 1 and Generation Unit 23)

FIG. 3A is a diagram showing an example of a functional block configuration of the system 1. The system 1 includes a storage unit 20, a display control unit 21, an input unit 22, and a generation unit 23. The storage unit 20 can be realized using the storage device 12 provided in the system 1. The processor 11 of the system 1 executes a program stored in the storage device 12, whereby the display control unit 21, the input unit 22, and the generation unit 23 are provided. The program may be provided over a network or stored in a storage medium. The storage medium storing the program may be a non-transitory computer-readable medium. The non-transitory storage medium is not particularly limited, but may be, for example, a storage medium such as a USB memory or a CD-ROM.

### (2-4-1. Configuration of Storage Unit 20)

The storage unit 20 includes various databases (DB). The storage unit 20 stores, as an example, an account DB, an image DB, a region DB, an annotation DB, a label DB, and a training data DB. An identification ID is assigned to stored data in each DB, and mutually related data can be cross-referenced.

The account DB stores a user ID, a user detail information, and a login history of each user. The user detail information may include an email address, etc., may include a name, etc. associated with an account (a nickname, an avatar icon, etc.), and/or may include more personal and specific information such as a name and an affiliation.

The image DB stores one or more medical images (for example, pathology images) used for generating the training data. The region DB stores information for identifying a region image in each medical image. As an example, the region DB stores, together with a region image ID, a medical image ID of a setting destination of the region image, and an image coordinate data. The image coordinate data defines which range of the medical image each region image occupies.

The annotation DB stores, together with an annotation ID, an annotation information for distinguishing each annotation performed using the system 1 from each other. The annotation information is preferably information that can identify for which medical image, when, by whom, and what type of annotation was performed. The annotation information can preferably store "annotation subject information". The annotation subject information is an identification information for identifying "who performed the annotation (that is, an annotation subject)". The annotation subject information may be, for example, a user ID (for example, a login account), or may be, for example, any one or more pieces of information in the user detail information stored in the account DB. The annotation information may include a disclosure availability of the annotation (that is, whether it may be disclosed on the selection screen 27 of another person with a different user ID), and may include a disclosure condition of the annotation (that is, under what conditions it is disclosed when disclosing to another person).

The label DB stores information of a label associated with a region image, which is an image of a region, by each annotation, in association with the annotation ID. The label DB stores each label ID and a label detail information in association with each other. The label detail information includes a classification type and a classification content of a label (for example, a type is a tumor classification, and a label classification content is a tumor, etc.).

The training data DB stores the generated training data. A user can download any training data by referring to the training data DB.

It is preferable that at least the image DB, the region image ID, the annotation ID, and the label ID are associated with each other. Thereby, by designating the annotation ID, a content of any annotation (that is, a medical image, a region image, a label, etc.) can be easily called from the annotation DB. Note that the above data management and DB configuration are merely examples. Any data management and DB configuration can be adopted according to actual specifications of hardware and/or software, etc. Note that when management is not performed on an annotation basis, the annotation DB can be omitted.

### (2-4-2. Configuration of Other Functional Units)

The display control unit 21 executes a display control to cause the display device 34 of the user terminal 3 to display various screens. For example, the display control unit 21 may be able to display a work screen 26 (see FIG. 4) in which a grid (boundary lines of a region) is superimposed on a medical image. A callout extending from the work screen 26 in FIG. 4 illustrates a screen in which a medical image Q is divided into four by a grid. In practice, a larger medical image Q is partitioned by the grid, and a dozen by a dozen region images are displayed on the work screen 26. A region image ID is associated with each region image. In the example of FIG. 4, one cell in the upper left of the medical image Q is also referred to as a region image Q₁. On the other hand, the display control unit 21 may also be able to display a designated frame superimposed on the medical image Q.

The input unit 22 accepts various inputs from the operation device 38 of the user terminal 3. For example, the input unit 22 accepts a label input operation (that is, an operation of the operation device 38 when a user performs an annotation). The label input operation is an input operation of which label to associate with each of a plurality of images of regions (region images). The input unit 22 stores a label associated with each region image in the label DB. The input unit 22 may, for example, store a region image ID that uniquely identifies each region image and a label associated with each region image in the label DB in association with each other. The input of the label may be performed, for example, by accepting a selection of a region image for which a label is to be input from each region image displayed on the screen, and accepting a designation of a label to be associated with the region image.

The generation unit 23 generates training data for training the learning model. For example, the generation unit 23 acquires a region image ID and a label from the label DB, and generates a training data image by cropping a region image corresponding to the region image ID from a medical image stored in the image DB. The generation unit 23 generates the training data by combining a training data image data and a label stored in association with the region image ID.

FIG. 3B is a functional block configuration of the generation unit 23. The generation unit 23 includes a data acquisition unit 23a, a label determination unit 23b, and a training data configuration unit 23c. Operations of each functional block will be described in the processing flows of FIG. 4 and FIG. 5.

### (2-5. Processing Flow)

FIGS. 4 to 6 and 8 are each a specific example of a processing flow of the first embodiment, and provide a program and a generation method for generating training data. In the processing flows of FIG. 4 and FIG. 5, a grid-method annotation is adopted. In the processing flows of FIG. 6 and FIG. 8, a designated-frame-method annotation is adopted.

In the first embodiment, a generation process is caused to be executed by a computer (the system 1) (see S103 to S106 of FIG. 4, or S103, S115, and S106 of FIG. 5). This generation process causes the computer (the system 1), by a manual operation or an automatic process, to perform an association between a region image and a label based on a plurality of pieces of information associated with the region image, thereby generating the training data. As will be described later, in the first embodiment, a first information and a second information are associated with each region image. Since a label association of the region image can be performed based on the plurality of pieces of information associated with the region image, the training data can be generated with high convenience.

The "plurality of pieces of information" mentioned here adopts different information in the first embodiment and the second embodiment. In the first embodiment, the "plurality of pieces of information" above includes a first information and a second information, and a first label and a second label are adopted as this first and second information. Specifically, a process of acquiring the first label by a first annotation and the second label by a second annotation is caused to be executed by the computer (the system 1). This process is comprehensively described in step S100 in FIG. 4, and will be detailed below.

In the first embodiment, a plurality of labels associated with a region image in a medical image may have the same classification content. Such a case is also simply referred to as a "label match", and a plurality of labels that are associated with a region image and have the same classification content are also referred to as "matching labels". On the other hand, there may be cases where labels with different classification contents are associated with a region image, that is, where judgments by a plurality of annotations are divided. Such a case is also simply referred to as a "label mismatch", and a plurality of labels that are associated with a region image and have different classification contents are also referred to as "mismatching labels".

### (2-5-1. Common Processing Step S100)

In the processing flows of FIGS. 4 to 6 and 8, first, a user U001 who has logged into the system 1 (S300a) receives a work screen 26 from the system 1 (the display control unit 21) (S101a). The work screen 26 is a GUI for an annotation work, that is, a label input operation. When the user U001 performs a "first annotation" via this work screen 26, a label input operation for each region image is performed (S301a). When, for example, a first label La₁ and a grid region image Q₁ are associated by an operation of the user U001 in step S301a, the system 1 (the input unit 22) registers the association in the label DB (S102a). A similar label association is also performed for other grid region images Q₂, Q₃, ... of the medical image Q.

Similarly, a user U002 who has logged into the system 1 (S300b) receives the work screen 26 from the system 1 (S101b). When the user U002 performs a "second annotation" via this work screen 26, a label input operation for each region image is performed (S301b). In step S301b, for example, a second label Lb₁ and the region image Q₁ are associated by an operation of the user U002, and the system 1 registers the association in the label DB (S102b). A similar label association is also performed for other grid region images Q₂, Q₃, ... of the medical image Q.

In the case of the processing flows of FIG. 4 and FIG. 5, a label is input to a grid region image in step S100. In the case of the processing flows of FIG. 6 and FIG. 8, in step S100, a designated frame region image is set by a designated frame, and a label is input to this designated frame region image.

Here, for a region image for which a label input has been completed in each of steps S301a and S301b, information indicating a classification content of the input label can be displayed superimposed on the region image. In the first embodiment, as an example of the label La₁ or Lb₁, a label classification content is displayed with the characters "T" and "N" (S301a, S301b). For a region to which a label indicating that a region image is an image suspected of having tumor cells is assigned, the character "T" is displayed. For a region image to which a label indicating that the region is not an image suspected of having tumor cells is assigned, the character "N" is displayed. The region image Q₁ in FIG. 4 illustrates a case where the label contents assigned in the first and second annotations are different, and the judgments by the users U001 and U002 are divided.

In FIG. 4, one grid region image Q₁ is exemplarily illustrated for convenience of explanation. However, in reality, an annotation is performed on a large number of grid region images Qₖ (that is, Q₁, Q₂, ..., Qₙ). k is a region image identifier introduced for convenience of explanation, and specifically, k = 1, 2, ..., n (where n is an integer), and corresponds to a region image ID. By the first annotation, first labels La₁ to Laₙ are associated with each of one or more region images Q₁ to Qₙ. By the second annotation, second labels Lb₁ to Lbₙ are associated with each of the one or more region images Q₁ to Qₙ. Hereinafter, for the purpose of referring to an arbitrary region image or label, it may be described as a region image Qₖ, a first label Laₖ, or a second label Lbₖ.

### (2-5-2. First Processing Flow Example)

FIG. 4 is a first example (a manual determination process) of a processing flow representing a control operation of the first embodiment. The example of FIG. 4 performs a label determination by accepting a user's manual selection operation. The first and second labels by the first and second annotations are already registered in the label DB by the aforementioned step S100. At this stage, a user who wants the training data (the user U002 in the example of FIG. 4) issues a training data generation command to the system 1 via the operation device 38 (S302). In this training data generation command, an input data necessary for a current training data generation can be designated. As an example, as a target for adoption of the training data, a medical image ID may be specifiable, a range of region image IDs may be specifiable, and an annotation call mode may be settable.

In response to the training data generation command (S302), the system 1 starts a generation process of the training data (S103). In the example of FIG. 4, the generation process includes steps S103 to S106. First, the data acquisition unit 23a (see FIG. 3B) of the generation unit 23 refers to each DB of the storage unit 20. The data acquisition unit 23a reads out each region image Qₖ to be included in the current training data according to the content of the training data generation command, and on the other hand, searches the label DB by a region image ID to read out each label associated with each region image Qₖ. The labels read out here include the first and second labels Laₖ and Lbₖ.

The data acquisition unit 23a performs a different data acquisition process depending on the annotation call mode. The annotation call mode can adopt, as an example, a designated call mode, an all call mode, or a stratified call mode, and a user may be able to selectively use two or more of these.

In the designated call mode, a desired annotation is designated by an annotation ID or the like, and a region image ID and a label ID of the designated annotation are referred to. In the all call mode, all annotations associated with a medical image at the time of calling are referred to, and a region image ID and a label ID of all the annotations are referred to.

In the stratified call mode, only an annotation corresponding to a specified condition is extracted, and a region image ID and a label ID of the extracted annotation are referred to. Various types of stratification can be set. For example, it may be a stratification based on whether annotation subject information meets a predetermined condition. For example, only an annotation with a performance value equal to or higher than a predetermined value may be extracted, whereby only an annotation with a high performance and reliability can be used. For example, a period designation may be possible as one of the call conditions, and only an annotation that has been registered (specifically, a new registration or a final edit registration) within a designated period may be extracted. As another example, an index value may be used, and for example, only an annotation in which a summary statistic of all labels within the annotation (for example, an average index value or a median value of the index values of all labels) is equal to or higher than a predetermined threshold may be extracted, whereby only an annotation created by an annotation subject with a certain degree of confidence can be used.

Next, the label determination unit 23b executes a label determination process (S104, S105). The label determination unit 23b generates a selection screen 27 based on the data acquired in step S103 and transmits it to the user terminal 3 (S104). The selection screen 27 is a GUI for causing the user U002 to select which of a plurality of labels associated with each region image Qₖ should be a determined label. In FIG. 4, as an example, the system 1 accepts an operation to select either the first label La₁ or the second label Lb₁ in the region image Q₁. For example, the user U002 can determine a desired label by clicking with a mouse pointer P or the like (S303). In reality, a plurality of region images Qₖ may be comprehensively arranged on the selection screen 27. In the example of FIG. 4, it is assumed that the second label Lb₁ has been clicked. The label determination unit 23b determines the label selected by the user (the second label Lb₁ in this example) as a determined label, and associates this determined label with the region image Q₁. Such an operation and process are repeated for each region image Qₖ necessary for the training data.

When the necessary region images Qₖ and determined labels are complete, the process proceeds to step S106. In step S106, the training data configuration unit 23c of the generation unit 23 crops the medical image Q according to the grid to generate a training data image of the same size as the region image by the grid. The training data configuration unit 23c configures the training data in which the training data image and the label are associated, and registers it in the DB (S106). The user U001 can arbitrarily download the training data.

As described above, the processing flow of FIG. 4 causes the computer (the system 1) to execute the generation process. The generation process causes the computer (the system 1), according to a manual selection operation (S303) received via the operation device 38, to determine a determined label for each region image Qₖ based on a plurality of labels Laₖ and Lbₖ associated with the region image Qₖ (S105), and to associate the determined label with the region image, thereby generating the training data from the medical image (S106). A mechanism that allows a determined label of each region image Qₖ to be manually determined based on a label (for example, La₁, Lb₁) by each of the first annotation (S301a) and the second annotation (S301b) can be provided in a form that is highly convenient for a user. The training data can be efficiently generated while taking a plurality of annotations into consideration, thereby improving convenience.

Note that in step S104, a user's label selection operation may be accepted for each region image Qₖ without distinguishing between a label match and a label mismatch, but as a modification, a batch determination process may be provided. The batch determination process is a process of, based on a user's instruction (for example, a click of a predetermined button), collectively determining a classification content of the matching label for each region image with a label match.

Note that in FIG. 4, a training data generation command (S302) is transmitted to the system 1 by a second user U002 who has previously performed an annotation work. However, this is just an example and is not limited to this. As another example, another third user may perform only a determination operation (S303) without performing a label input operation (S301a, S301b) for the medical image Q. Also in this case, a label selection of each region image by the determination operation (S303) corresponds to an annotation work.

### (2-5-3. Second Processing Flow Example)

FIG. 5 is a second example (an automatic determination process) of a processing flow representing a control operation of the first embodiment. In FIG. 5, a computer (which is the system 1, specifically the label determination unit 23b) automatically executes a label determination process (S115) according to a preset rule. This point is a difference from the example of FIG. 4. Other steps S100 (S101a to S102b), S103, and S106 are the same as in the flow of FIG. 4.

After step S103, the label determination unit 23b executes a label determination process (S115).

A label determination process at the time of a label match may be, for example, as follows. A case is assumed where there is a region image Qₖ in which the first and second labels Laₖ and Lbₖ show the same result as each other. A process of making a determined label the same content as the first and second labels Laₖ and Lbₖ is caused to be executed by the computer (the system 1). Specifically, when it is assumed that a first and a second label La₂ and Lb₂ (both not shown) associated with a region image Q₂ are both "T" (that is, a tumor), in this case, a determined label of the region image Q₂ is set to "T".

On the other hand, a label determination process at the time of a label mismatch may be, for example, as follows. A case is assumed where there is a region image Qₖ in which the first and second labels Laₖ and Lbₖ show different results from each other. In this case, a process preset as the label determination process is executed. As an example, a process of setting a result of one of the first and second labels Laₖ and Lbₖ as a determined label according to a predetermined rule may be caused to be executed by the computer (the system 1). To give a specific example, a case is assumed where the first and second labels La₁ and Lb₁ associated with the region image Q₁ are different, being "T" and "N", as in the example of FIG. 5. In this case, the label determination unit 23b may determine a determined label based on information of an annotation subject (the users U001 and U002 in the first embodiment), as an example. An example of the information of the annotation subject is a " performance value". As an example, the label determination unit 23b may read out a performance value of the user U001 who assigned the first label La₁ and a performance value of the user U002 who assigned the second label Lb₁ from the storage unit 20, and compare those performance values. As a result of the comparison, a label assigned by a user with a relatively higher performance value may be set as the determined label. As another example, when there is a region image Qₖ with a label mismatch, a process of excluding the region image Qₖ from a target of the training data may be caused to be executed by the computer (the system 1). As yet another example, a manual operation selection process (for example, S104 and S105 of FIG. 4 can be used) may be adopted for a region image Qₖ with a label mismatch, and thereby a process of accepting a label selection from a user may be caused to be executed by the computer (the system 1).

As described above, the processing flow of FIG. 5 causes a computer to execute the generation process. The generation process causes the computer (the system 1), according to an automatic process of the computer (the system 1) (the label determination process of S115), to determine a determined label for each region image Qₖ based on a plurality of labels (the first and second labels Laₖ and Lbₖ) associated with the region image Qₖ, and to associate the determined label with the region image Qₖ, thereby generating the training data from the medical image. A determined label of each region image can be automatically determined based on a label by each of the first and second annotations. The training data taking a plurality of annotations into consideration can be generated efficiently and with a small work burden, thereby improving convenience.

### (2-5-4. Third Processing Flow Example)

The processing flow of FIG. 6 is similar to the processing flow of FIG. 4 in many respects. However, as a major difference, the example of FIG. 6 adopts a designated-frame-method annotation. In step S101a, the work screen 26 displays the medical image Q without a grid. In steps S301a and S301b, the users U001 and U002 set designated frame region images Q₁ₐ and Q_{1b} in the medical image Q using a designated frame via the work screen 26, and input labels La₁ and Lb₁.

In a selection screen presentation process (S104), the selection screen 27 is generated so that the designated frame region images Q₁ₐ and Q_{1b} and the labels La₁ and Lb₁ are displayed superimposed on the medical image Q. As an example of a determination operation (S303), the user U002 aligns the mouse pointer P with either of the designated frames of the designated frame region images Q₁ₐ and Q_{1b} and performs a click operation. By this operation, either a set of the designated frame region image Q₁ₐ and the label La₁, or a set of the designated frame region image Q_{1b} and the label Lb₁ is adopted. A selection operation by the user U002 is similarly performed for all the designated frames associated with the medical image Q.

Thereafter, in step S106, a process for configuring the training data is executed. The training data configuration unit 23c can crop the medical image Q according to the designated frame to generate a training data image of the same size as the region image by the designated frame.

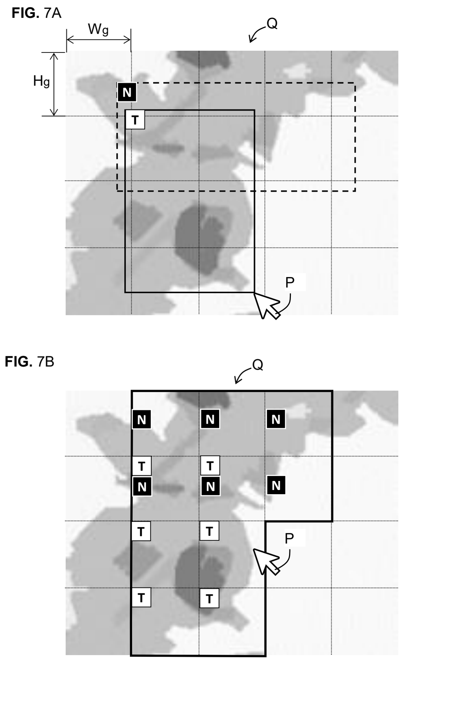

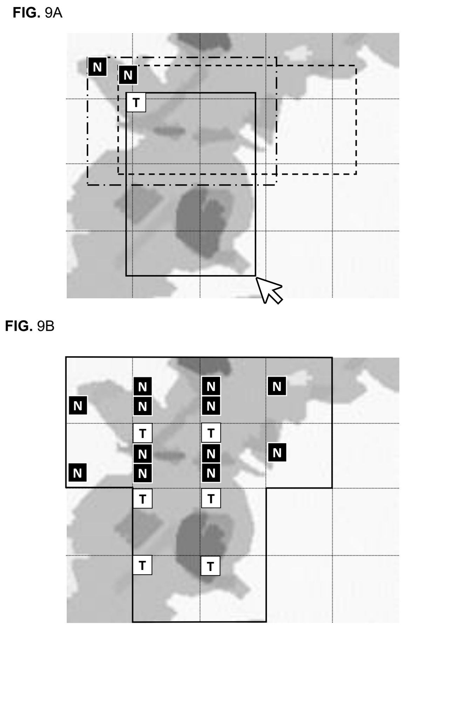

As a modification, the computer (the system 1) may be caused to execute a region image conversion process described below in step S104. The "region image conversion" mentioned here refers to performing a conversion between an image of a designated frame region and an image of a grid region. First, as illustrated in FIGS. 7A and 7B, a grid is superimposed on a designated frame. For a region image conversion, as an example, a label of a designated frame image is associated with each region image (width Wg x height Hg) defined by the grid of FIG. 7A within a range covered by the designated frame. As an example, when an overlapping area between a designated frame region and a grid region is equal to or greater than a "predetermined overlap amount", the same label as the designated frame region image may be associated with a grid region image. The "predetermined overlap amount" is, for example, 20%, 30%, 40%, 50%, 60%, 70%, 80%, 90%, or 100% of an area of the grid region, and may be within a range between any two of the numerical values exemplified here. When the predetermined overlap amount is 100%, a certain grid region and a designated frame region completely overlap. In FIG. 7B, which is after the region image conversion, a region group with T labels of 3 rows and 2 columns and a region group with N labels of 2 rows and 3 columns are generated. Two grid region images in FIG. 7B are AND region images of two designated frames, and both a T label and an N label are associated with them. The user U002 may perform the same determination operation (S303) as in the flow of FIG. 4 for each grid region image in FIG. 7B. Thereafter, in step S106, a training data image can be generated according to the grid of FIG. 7B. Note that a case where there are three designated frames is illustrated in FIGS. 9A and 9B, so please refer to this as well.

### (2-5-5. Fourth Processing Flow Example)

The processing flow of FIG. 8 is similar to the processing flows of FIG. 5 and FIG. 6 in many respects. A difference from the processing flow of FIG. 5 is that the example of FIG. 8 adopts a designated-frame-method annotation. A difference from the processing flow of FIG. 6 is that the example of FIG. 8 performs an automatic label determination process. Except for these differences, the processing flow of FIG. 8 is the same as the processing flows of FIG. 5 and FIG. 6.

In the flow of FIG. 8, in a label determination process (S125), a label of a designated frame region image set in the medical image Q is determined. The process of step S125 can use the process of step S115 by compensating for a difference between a grid region and a designated frame region. In step S125, an automatic selection of a designated frame may be performed without performing a region image conversion. In this case, as an example, a designated frame image and a label with a higher performance value may be automatically adopted. Thereafter, in step S106, a training data image can be generated according to the designated frame image.

In the process of step S125, the generation unit 23 may perform a region image conversion. After the region image conversion, a state in which a label is associated with a grid region image is created, as shown in FIG. 7B. After this, the process of step S115 can be similarly applied. Thereafter, in step S106, a training data image can be generated according to the grid of FIG. 7B.

When a region image conversion is applied, as an example, the computer (the system 1) may be caused to execute a label determination process described below in step S125. In the region image conversion, an overlap between a designated frame region image and a grid region image may differ for each designated frame. For example, in FIG. 7B, T and N labels are associated with two grid region images, but referring to FIG. 7A, in the grid region image on the right side of the figure, a designated frame region image with an N label covers the entire grid region image, whereas a designated frame region image with a T label does not extend to the entire grid region image. Such a difference in overlap between a grid region image and a designated frame region image may be included in the label determination process. As an example, when there is a difference in an overlap degree with a grid region among a plurality of designated frame region images, a label of a designated frame region image having a maximum overlap area may be adopted as a label of that grid region image. As another example, as an example, when a difference amount of an overlap area between a designated frame region image with a maximum overlap area and another designated region image is equal to or greater than a threshold, a label of the designated frame region image with the larger maximum area may be adopted as a label of that grid region image, and when the difference amount of the overlap area is less than the threshold, a label determination based on the overlap area may not be adopted and a determination process may be executed based on another criterion. Thereby, when there is a designated frame region image whose overlap area is not so large, an importance can be lowered. The process described here can be similarly adopted even when there are three designated frame images as in FIGS. 9A and 9B, or when there are four or more designated frame images.

### (2-6. Modifications, etc., of First Embodiment)

The first embodiment can be variously modified at least as described below.

### (2-6-1. Index Value)

As a modification, an "index value" may be incorporated into the control of the first embodiment. The index value is a parameter that can be associated with each of a plurality of pieces of information. The index value represents a certainty of a content of each of the plurality of pieces of information. There is an advantage that more accurate training data can be generated based on the index value. The plurality of pieces of information (a plurality of labels in the first embodiment) may be the index value itself, or may comprise the index value. Hereinafter, a specific description will be given.

### (2-6-1-1. Example of Adopting Index Value)

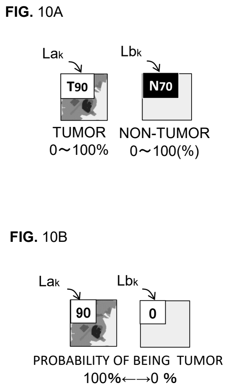

FIG. 10A is an example of a label image in which a label comprises an index value, and FIG. 10B is an example of a label image in which a label is an index value. As in FIGS. 10A and 10B, a first label Laₖ and/or a second label Lbₖ associated with a region image as an annotation target may comprise an index value or be an index value. The index value in this example represents how certain a label classification result is. The index value may be manually inputtable by a user, for example, during an annotation work on the work screen 26. The index value can be adopted in an annotation of either the grid method or the designated frame method. For example, the first label Laₖ in FIG. 10A is described as "T90", which means that a label classification content is "T" (a tumor) and its index value (a certainty) is 90(%). For example, a second label Lbₖ in FIG. 10A is described as "N70", which means that a label classification content is "N" (a non-tumor) and its index value (a certainty) is 70(%). The index value may be inputtable by the users U001 and U002 by a manual operation in steps S301a and S302b of FIGS. 4 and 5. The index value may be arbitrarily settable by a user with an arbitrary step width (for example, a constant width such as a 1% step or a 5% step), or alternatively, the index value may be presented as, for example, a plurality of options (for example, an arbitrary number of levels such as three levels or four levels), from which the user may be able to select a desired index value. For example, as a three-level index value, "High" (for example, 70% or more), "Medium" (for example, 31% to 69%), and "Low" (for example, 30% or less) may be used. There is an advantage that a convenience of a user's index value input is improved.

### (2-6-1-2. Application Example to Processing Flow of FIG. 4 or FIG. 6)

When adopting an index value in the example of FIG. 4 or FIG. 6 (a manual operation), a label with an index value may be associated with a region image as in FIGS. 10A and 10B on the selection screen 27. A user can visually recognize and compare the index value of each label. The user can determine a determined label of each individual region image Qₖ while also taking into account a level of the index value. Note that among a plurality of labels, a label with the highest index value may be displayed prominently. A way of making it stand out may be, for example, attaching an arbitrary badge to a label, a highlight display (an emphasis display), or displaying the label itself relatively large.

### (1-6-1-3. Application Example to Processing Flow of FIG. 5 or FIG. 8)

In the case of the example of FIG. 5 or FIG. 8 (an automatic process), in step S115, a label determination process based on an index value may be caused to be executed by a computer. As an example, a label with a maximum index value among a plurality of labels may be adopted as a determined label. As another example, a difference of an index value may be calculated between mismatching labels, and when the difference is equal to or greater than a predetermined reference value, a label with a relatively larger reference value may be adopted as a determined label. On the other hand, when a difference of an index value between mismatching labels is less than the reference value, a region image with which the label is associated may not be included in the training data, or a user may be allowed to decide which label to adopt for the region image by a manual operation selection process (for example, S104 and S105 of FIG. 4 can be used). By integrating a plurality of annotation results while including an index value, more accurate training data can be automatically generated.

### (2-6-2. Number of "Plurality of Pieces of Information")

### (2-6-2-1. Regarding Number of Annotations and Labels)

In the first embodiment, as illustrated in FIGS. 4 to 9, two pieces of information, a first information and a second information (a first label and a second label), are adopted as an example of a "plurality of pieces of information". However, it should be noted that this only defines a minimum configuration, and an arbitrary number of three or more pieces of information (labels) can be added. For example, an association between a region image and a label may be performed based on a first to a third information. For example, a third label may be associated with a region image Qₖ by a third annotation of another third user, and this third label may be acquired together with the first and second labels in step S103. A process of determining a determined label based on the first to third labels for each region image Qₖ (step S104, S105 of FIG. 4 or S115 of FIG. 5) may be caused to be executed by the computer (the system 1), and a process of generating training data in which the determined label and the region image are associated (S106) may be caused to be executed by the computer (the system 1). In the first embodiment, the description "determine a determined label based on the first to third labels" includes the meaning of "determine a determined label from options ("T" or "N") presented by the first to third labels".

In the above example, for convenience, a first to a third annotation are exemplified, and a first to a third label can be adopted as the "plurality of pieces of information". However, there is no upper limit to the number of the "plurality of pieces of information" that can be adopted in the first embodiment. An arbitrary number of annotations by an arbitrary number of users may be adopted in the processing flows of FIGS. 4 to 6 and 8. When a number of annotations that can be adopted in the processing flow of FIG. 4 or FIG. 5 in the system 1 is "Z", a value of Z is, for example, 2 to 100, for example, 3 to 20, and for example, 4 to 10. The value of Z is, specifically, for example, 2, 3, 4, 5, 6, 7, 8, 9, 10, 15, 20, 30, 40, 50, 75, or 100, and may be within a range of any two of the numerical values exemplified here.

The system 1 of the embodiment may be used by a limited number of members, for example, only by members within a certain company, laboratory, or research group. However, the present invention is not limited to this, and the system 1 may be able to accept user account registrations from members of many more various research institutions, universities, or companies, may be accessible to users in all regions of the world, and for that purpose, may be widely disclosed to the public through the communication network 2. By doing so, there is an advantage that annotation data can be collected from many more various users. However, in that case, not all users are necessarily favorable to disclosing their own user information. In order to address this point, the system 1 of the embodiment includes a restriction function regarding an annotation disclosure, and/or a "display/non-display function of annotation subject information" to be described later.

The system 1 may adopt all labels associated with a region image Qₖ and execute a determination process (S104 and S105 of FIGS. 4 and 6, S115 of FIG. 5, and S125 of FIG. 8), but is not limited to this. For example, in a training data generation command (S302), a user may be able to designate a label group to be adopted in a generation process of the training data, that is, may be able to designate which annotation performed by whom and when to adopt. For example, the system 1 may display a user ID of each label associated with a region image ID to be a training data generation target on the user terminal 3 in response to the training data generation command. A selection designation of a user ID may be accepted at the user terminal 3, and the designated user ID may be transmitted to the system 1.

### (2-6-2-2. Application Example to Processing Flow of FIG. 4 or FIG. 6)

As an example, in the selection screen 27 of the processing flow of FIG. 4, a process of displaying a first to a third label on one grid region image Qₖ may be caused to be executed by a computer (the system 1, specifically the display control unit 21). A user can select one label from the first to the third labels. The same applies when four or more pieces of information are adopted.

As another example, the selection screen 27 may be presented in a form in which a difference in the number of labels belonging to one (for example, "T") and the other (for example, "N") of a label classification content among three or more labels is visible to a user. In this case, on the selection screen 27, one "T" label and one "N" label may be displayed superimposed on each grid region image Qₖ, and a difference in the number of labels may be expressed on each label image. For example, on the selection screen 27, a number of "T" labels and a number of "N" labels may be respectively presented with numerical values or the like (the numerical values may be displayed superimposed on a grid region image), or a label with a larger number may be displayed prominently. A way of making it stand out may be the same as the example described in the aforementioned "1-6-1-2. Application Example to Processing Flow of FIG. 4".

As another example, in the selection screen 27 of the processing flow of FIG. 6, three designated frame region images may each have a first to a third label (see FIG. 9A). As another example, when performing a region image conversion in the processing flow of FIG. 6, each application example of FIG. 4 described above can be adopted for a grid region image after the region image conversion (see FIG. 9B).

### (2-6-2-3. Application Example for the Processing Flow of FIG. 5 or FIG. 8)

As an example, when adopting the first to third information in the label determination process (S115) of FIG. 5, a computer (the system 1, specifically, a label determination unit 23b) may be made to execute a majority vote process. In the majority vote process, for example, the number of "T" labels and the number of "N" labels of the first to third labels may be counted for each region image, and for each region image, the one with the higher count between "T" and "N" may be adopted as a determined label. The majority vote process is similarly applicable when a region image transformation is performed in the processing flow of FIG. 8 (see FIG. 9B).

The majority vote process can also be adopted when using four or more pieces of information (that is, the first to fourth labels, etc.). However, when the number of "a plurality of pieces of information" is an even number, the disagreeing labels may be balanced at half each. In a case of a balance, as an example, the determined label may be decided using an annotation performance value. Specifically, for example, the classification content of the label with the maximum annotation performance value may be decided as the determined label. As another example in a case of a balance, a total value of the annotation performance values of users who assigned a "T" label and a total value of the annotation performance values of users who assigned an "N" label may be compared, and the label with the larger total value may be used as the determined label. As yet another example in a case of a balance, the region image with the label mismatch may be excluded from the training data. As a further example in a case of a balance, the computer (the system 1) may be made to execute a process of accepting a label selection from a user for the region image Qₖ with the label mismatch through a manual operation selection process (for example, S104 and S105 of FIG. 4 can be used).

As an example, when adopting three or more pieces of information (labels) in the label determination process (S115) of FIG. 5, the computer (the system 1, specifically, the label determination unit 23b) may be made to execute an arithmetic process based on an index value. The arithmetic based on the index value may be, for example, a four-rule calculation (e.g., addition, etc.), or a summary statistic calculation (e.g., mean value calculation or maximum value calculation). For example, among the first to third labels, a first added value may be obtained by adding the index values of "T" labels, a second added value may be obtained by adding the index values of "N" labels, and the label corresponding to the larger of the first and second added values may be adopted as the determined label. Alternatively, a first mean value and a second mean value may be obtained instead of the first and second added values, and the label corresponding to the larger of the first and second mean values may be adopted as the determined label. Alternatively, for example, the label with the maximum index value among the first to third labels may be adopted as the determined label. The same applies to four or more pieces of information (labels). The arithmetic process based on the index value is similarly applicable when the region image transformation is performed in the processing flow of FIG. 8 (see FIG. 9B).

### (2-6-2-3. Countermeasure for Occurrence of Missing Labels)

The plurality of region images Qₖ may include a region image in which a label is missing (i.e., an annotation omission). Specifically, for example, although N labels have been associated in many other (e.g., a majority of) region images, fewer than N labels may be associated or the number of labels may be zero in some region images. In this case, as an example, when at least one label is associated with the region image where a label is missing, a process of determining the determined label based on that at least one label may be performed. As another example, for a region image with a missing label and/or a zero number of labels, that region image may be excluded from the target of training data generation, or a label supplement input from the user may be prompted for that region image by an annotation reception process (for example, S101a, S301a, S102a of FIG. 4 can be used).

### (2-7. Disclosure/Non-disclosure of Annotation Subject Information)

An annotation subject information may be displayed on a selection screen 27. The annotation subject information is information that can identify who the annotation subject that performed the label input for each label is. The annotation subject information may be a user ID or the like, or it may be a user's name, affiliation, nickname, an avatar icon, or the like. However, on the other hand, the annotation subject information may not be displayed. As an example, the annotation subject information of an annotation called to the selection screen 27 may not be displayed. A user may be able to set "disclosable" or "non-disclosable" for the annotation. In the case of "disclosable," it may be displayed on the selection screen 27 conditionally (i.e., on the condition that the annotation subject information is not displayed). By not displaying the annotation subject information, the annotation subject can be kept confidential. Even if a user is not favorable to disclosing their own user information, there is an advantage that annotation data can be utilized while ensuring anonymity.

A subject that performs a first and a second annotation (an annotation subject) is not limited to a person and may also include a computer. When a computer performs an image detection process by a rule-based classification process or by a machine inference classification of a trained discrimination model, a classification result is associated with each region image, so this may be regarded as an annotation. The subjects of the plurality of annotations may be different or the same. A user ID for a computer may be set in an account DB, and an annotation subject ID specific to each computer may be assigned at the time of registration to a label DB.

### <3. Second Embodiment>

Among the second embodiment, the description of configurations that are the same as or similar to the first embodiment will be omitted or simplified. Hereinafter, the explanation will focus on the differences.

### (3-1. Functional Block Configuration of System 1 and Generation Unit 23)

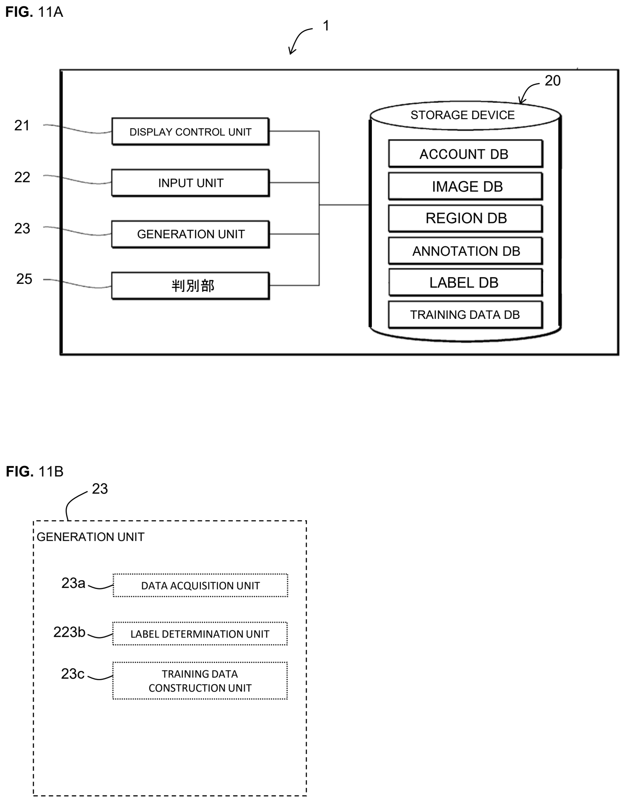

The system 1 of the second embodiment shown in FIG. 11A includes a storage unit 20, a display control unit 21, an input unit 22, a generation unit 23, and a discrimination unit 25. It differs from the system 1 of the first embodiment (see FIG. 3A) in that it includes the discrimination unit 25. The generation unit 23 of the second embodiment shown in FIG. 11B includes a data acquisition unit 23a, a label determination unit 223b, and a training data construction unit 23c. The second embodiment differs from the system 1 of the first embodiment (see FIG. 3A) in that it includes the label determination unit 223b that executes an automatic labeling process, instead of the label determination unit 23b that determines a label selected by a user as the determined label.

### (3-2. Description of Discrimination Unit 25)

The discrimination unit 25 is a trained discrimination model constructed with an arbitrary machine learning algorithm. There is no limitation on the machine learning algorithm for constructing the discrimination unit 25, and various known technologies can be adopted, for example, a neural network, a decision tree, a support vector machine, or a logistic regression analysis, etc., for example, an arbitrary deep learning algorithm (DNN), and for example, an arbitrary CNN model. The discrimination unit 25 has already completed its training using other training data. The discrimination unit 25 is constructed to be able to execute an image recognition process on input image data and to execute a machine inference to classify the input image data.

### (3-3. Processing Flow)

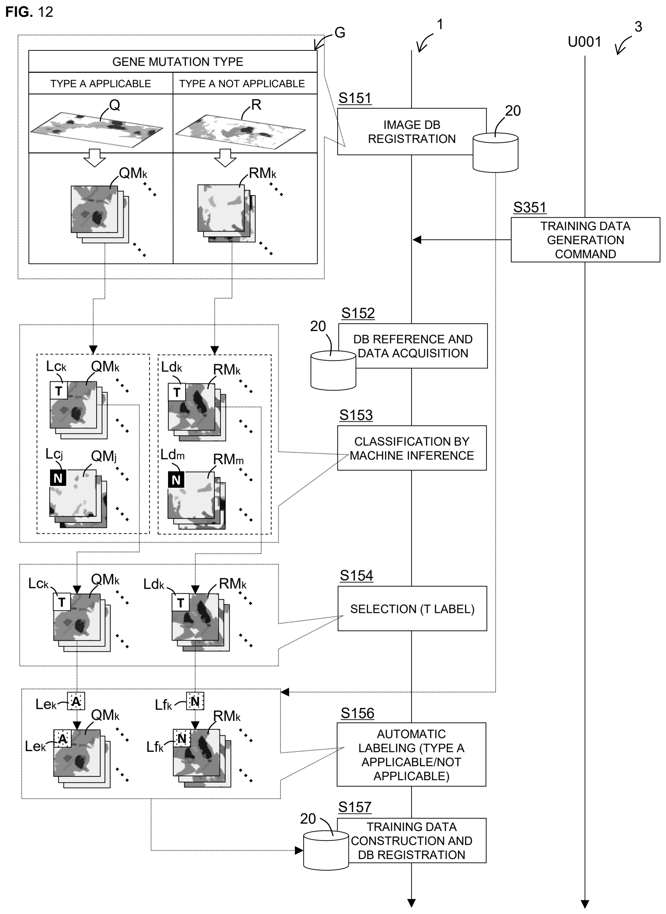

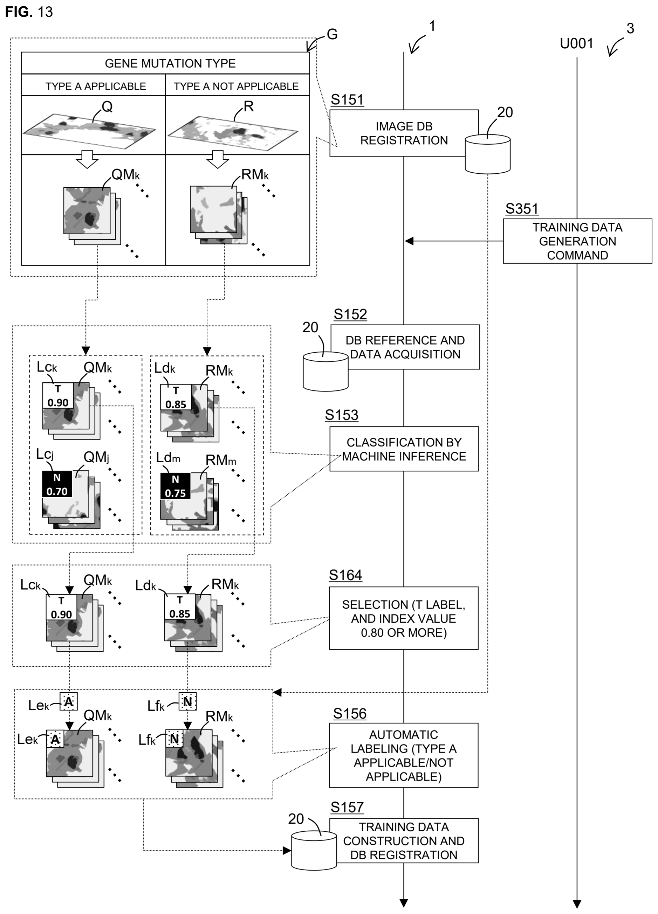

FIG. 12 and FIG. 13 are each a specific example of the processing flow of the second embodiment, and provide a program and a generation method for generating training data. In the second embodiment, a computer (the system 1) is made to execute a generation process different from that of the first embodiment (see S152 to S157 of FIG. 12, or S152, S153, S164, S156, and S157 of FIG. 13). The generation process of the second embodiment is an automatic process in which a computer (the system 1) is made to associate the region image with a label based on a plurality of pieces of information (first and second information) associated with the region image, thereby generating the training data. As will be described later, also in the second embodiment, the first and second information are associated with the region image for each region image. Since label association with the region image can be performed automatically based on the plurality of pieces of information associated with the region image, the training data can be generated with high convenience.

In the second embodiment, the aforementioned "a plurality of pieces of information" include the first and second information, "image-attached information G" is adopted as the first information (see FIG. 12), and "inference classification result by a trained discrimination model" is adopted as the second information (exemplified by labels Lcₖ and Ldₖ in FIG. 12). The image-attached information G is information attached to a medical image (the entirety of a medical image). Typical examples may include various kinds of medical information that describe the details of the medical image, or information of a patient from whom the medical image was acquired, and specific variations will be described later.

### (3-3-1. Common Processing Step S151)

As a common point in FIG. 12 and FIG. 13, a computer (the system 1) is made to execute a process of accepting input data including a plurality of region images (S151). In the second embodiment, the storage unit 20 stores a first medical image Q and a second medical image R. A plurality of region images QMₖ, RMₖ can be generated from each of the first medical image Q and the second medical image R. As an example, the plurality of region images QMₖ, RMₖ may be ones obtained by cropping each of the first and second medical images Q, R into a uniform size according to a grid. As another example, the plurality of region images QMₖ, RMₖ may be ones obtained by cropping each of the first and second medical images Q, R according to one or more designated frames set for each of the first and second medical images Q, R. The first and second medical images Q, R are associated with the first information (the image-attached information G). Here, a gene mutation type is adopted as an example of the image-attached information G. The gene mutation type of each medical image registered in an image DB is already known at the stage of registration to the storage unit 20 (the image DB) by various tests called a gene mutation test, a cancer panel test, or the like. The storage unit 20 stores each medical image in association with a gene mutation type (Type A applicable/not applicable, Type B applicable/not applicable, etc.).

In the second embodiment, as an example, the first information (the image-attached information G) is "gene mutation type," and a predetermined criterion (a labeling criterion) is "whether it corresponds to gene mutation type A." The first medical image Q is an image in which the first information satisfies the predetermined criterion. The second medical image R is an image in which the first information does not satisfy the criterion. In FIG. 12, the image-attached information G is schematically illustrated in a table, but in reality, the image-attached information G is stored in the image DB in association with an image ID.

Although it is expediently described as "gene mutation type A" here, it is assumed that what kind of gene mutation type A specifically is has been preset. As types of gene mutations, depending on a site of a cancer, there are, for example, an EGFR gene mutation, a HER2 gene amplification (ERBB2 copy number abnormality), a RAS gene mutation (KRAS and NRAS), a BRAF gene mutation (V600E or V600K), an ALK fusion gene, a ROS1 fusion gene, an NTRK fusion gene, high-frequency microsatellite instability (MSI-High), a BRCA gene mutation, or a tumor mutational burden (TMB), etc. It is sufficient to predefine what specifically, qualitatively and/or quantitatively, constitutes "gene mutation type A" among the various gene mutations, thereby making it possible to distinguish between applicable/not applicable for type A. Each type (A, B, C...) may be registered in the storage unit 20, and the applicability of each type may be selectively settable at the time of registering a medical image to the storage unit 20.

### (3-3-2. First Example of Processing Flow)

In the first example of the processing flow shown in FIG. 12, for example, when there is a training data generation command from a user U001 (S351), the system 1 starts a generation process of the training data. The processing flow of FIG. 12 first makes a computer (the system 1) execute a process of acquiring input data including a plurality of region images (S151). Specifically, each DB in the storage unit 20 is referenced, and the input data necessary for the generation of the training data this time is acquired (S152). In the example of FIG. 12, it is assumed that the user U001 specifies the first and second medical images Q, R, "gene mutation type A applicable/not applicable" is specified as the first information, "tumor/non-tumor discrimination" is specified as the second information, and the region images QMₖ, RMₖ generated from these are processed.

Note that it may be possible to specify an arbitrary medical image or an arbitrary region image, and/or it may be possible to arbitrarily specify the first and second information. This further improves convenience. For example, in step S151, the user U001 may be requested for several setting items. For example, the user U001 may be allowed to arbitrarily make at least one request among a specification request for a medical image or a region image, a specification request for the first information, and a specification request for the second information, and the content of the specification request may be transmitted from a user terminal 3 to the system 1.

In step S153, a computer (the system 1) is made to execute a process of having the trained discrimination model (the discrimination unit 25) perform an inference to classify the region images QMₖ, RMₖ of the input data. An inference classification result for each region image is "T" or "N," and this inference classification result is the "second information" of the second embodiment. Here, in FIG. 12, for the purpose of making the explanation easy to understand, a label Lcₖ for "T" (tumor) and a label Ldₖ for "N" (non-tumor) are associated with the region images QMₖ, RMₖ for convenience, but such labeling is not essential in the actual process (that is, label DB registration is unnecessary), and such an illustration does not limit the scope of the present invention. It is also acceptable that in step S153, each region image is simply classified into "T" or "N," and the classification result of each region image is merely provided to a subsequent process (S154).

In step S154, the label determination unit 223b executes a selection process for the region images QMₖ, RMₖ. In the flow example of FIG. 12, only region images to which a "T" label is assigned are extracted. Here, as an example, only the region image QMₖ and the region image RMₖ to which the "T" label is assigned are passed to the next step S156. Note that in FIG. 12, for convenience of explanation, region images to which an "N" label is assigned are exemplarily described as "QMⱼ, RMₘ," and labels associated with them are exemplarily described as "Lcⱼ, Ldₘ" (j and m are integers). These region images to which the "N" label is assigned are discarded in step S154. As a modification, it is also possible to extract only the region images to which the "N" label is assigned.

In step S156, the label determination unit 223b executes an automatic labeling process. That is, in the process of step S156, a computer (the system 1, specifically, the label determination unit 223b) is made to associate a label Leₖ with one or more region images (the region images QMₖ after selection in S154) that are generated from the first medical image Q and classified into a first classification group ("T") as a classification result, and to associate a label Lfₖ with one or more region images (the region images RMₖ after selection in S154) that are generated from the second medical image R and classified into the first classification group ("T") as a classification result. Information such as from which of the first and second medical images Q, R each region image QMₖ, RMₖ was generated is already stored in the image DB (see S151).

The label Leₖ indicates that the first information of the region image QMₖ satisfies the aforementioned predetermined criterion (corresponding to gene mutation type A). The label Lfₖ indicates that the first information of the region image RMₖ does not satisfy the criterion. In the generated training data, the labels Leₖ, Lfₖ become the correct answer data. In this way, the region images of the input data are classified by the trained discrimination model, and a label determined by the first information (the image-attached information G) can be automatically associated with the region images classified into the first group as the classification result (the second information). Note that a medical image or a region image whose applicability to the gene mutation type is unknown may be excluded at the stage of step S152.

In the second embodiment, the first information (the image-attached information G) can be set in various ways. When a medical image adopted in the second embodiment is a pathological image, the first information may be a gene mutation type, a TMB-H (high tumor mutational burden) type, a result of a biochemical analysis, or a prognostic prediction of a patient. A classification type and a classification content of a label determined by the first information in step S156 can be defined in various ways. The classification content of the label may be, for example, whether a gene mutation type corresponds to a predetermined type (e.g., type A), whether it corresponds to a TMB-H (high tumor mutational burden) type, whether a result of a biochemical analysis corresponds to a predetermined criterion, or whether a prognostic prediction of a patient is a predetermined reference value or more, etc. The prognostic prediction of the patient may be, for example, whether a life prognosis is a predetermined period or longer, etc. The first information may be a patient's age, sex, medical history, a result of an immunohistochemical analysis, or a lifestyle. These various classification types and classification contents may be registered in the storage unit 20 in advance, and may be selectively settable at the time of registering a medical image to the storage unit 20.

In step S157, a computer (the system 1) is made to execute a process of generating the training data with the region images and labels associated in step S156. The generated training data is registered in a training data DB, and the user U001 can arbitrarily download it.

As described above, the processing flow of FIG. 12 makes a computer (the system 1) execute the generation process. In this generation process, a computer (the system 1) is made to associate a label determined by the first information (applicability of gene mutation type A: S151) (see labels Leₖ, Lfₖ in S156 of FIG. 12) with region images selected based on the second information (tumor/non-tumor inference classification result: S153) (see region images QMₖ, RMₖ in S154) to generate the training data. Training data, for which label determination by the first information and selection by the second information have been applied to region images, can be automatically generated.

### (3-3-3. Second Example of Processing Flow)

FIG. 13 is a second example of a processing flow representing a control operation of the second embodiment (a process using an index value). Specifically, the difference between the respective flows of FIG. 12 and FIG. 13 is that step S154 and step S164 are replaced. The other steps S151 to S153, S156, and S157 are the same as in the flow of FIG. 12.

In the second example of the processing flow shown in FIG. 13, it should be noted that the discrimination unit 25 outputs a probability value in step S153. Some known image recognition models based on machine learning algorithms can output the certainty (that is, a probability or a degree of matching) of a classification result from image recognition. In the example of FIG. 13, it is assumed that the discrimination unit 25 is a neural network model constructed with a deep learning algorithm. An output layer of the neural network outputs, as an inference result, a probability value indicating the probability with which the input image data belongs to each classification result. In the example of FIG. 13, this probability value is used as the index value. By the inference in step S153, the trained discrimination model (the discrimination unit 25) outputs a probability value representing the certainty of the classification results for the region images QMₖ, RMₖ.

In step S164, a computer (which is the system 1, and specifically, the label determination unit 223b) executes a selection process for the region images QMₖ, RMₖ. Specifically, in the flow example of FIG. 13, the computer (the system 1) is made to extract only the region images for which the classification result of an inference classification process (S153) is "T" (tumor) and for which an index value (the probability value) is inferred to be a predetermined value or higher. In the example of FIG. 13, the predetermined value is 0.80.

This predetermined value is, for example, 0.70 to 1.00, and may be, for example, 0.80 to 1.00. This predetermined value may specifically be, for example, 0.50, 0.55, 0.60, 0.65, 0.70, 0.75, 0.80, 0.85, 0.90, or 0.95, and may be within a range between any two of the values exemplified here. The numerical values exemplified here may also be expressed as percentages after converting 1.00 to 100%.

Thereafter, similarly to the processing flow of FIG. 12, an automatic labeling process (S156) and a training data construction process (S157) are executed. By performing automatic selection using the probability value obtained as the inference result, training data with higher accuracy can be automatically generated.

### (3-4. Modifications, etc., of the Second Embodiment)

In the second embodiment, the discrimination unit 25 is built into the system 1, but this is merely an example and is not limited to such a form. The discrimination unit 25 does not have to be built into the system 1. The process of step S153 may be executed by collaborating with an arbitrary external system that has a trained discrimination model and using this external system as the discrimination unit 25.

### <4. Others>

### (4-1. Criteria for Substantial Identity of Images, and DB Management)

In the embodiments, the following criterion of "substantial identity" can be adopted for the identity between a plurality of medical images. As an example of a case where two medical images are substantially identical, at least the following first or second situation can be cited. In a first situation, the two medical images are completely identical and have the same image data. In a second situation, even if there is a difference between the medical images (for example, a difference in image data and/or image ID), that difference can be regarded as identical for annotation purposes.

For example, it is assumed that a certain medical image is acquired by imaging the same medical image object. It is assumed that a new medical image is generated by duplicating the medical image data, changing an image format after duplication (e.g., saving in a compressed format, etc.), or applying image processing (resizing, changing brightness, contrast, saturation, color tone, etc.) after duplication. The original medical image and the new medical image are substantially identical, even if there are differences in the image ID, image data, etc. This is because the imaging object of the medical images is the same, and the difference can be ignored for annotation purposes. This is also the same for region images, and the criterion of substantial identity can also be adopted for region images generated from substantially identical medical images.

Alternatively, for example, a mechanism called a "virtual slide" may be used. In a virtual slide, the same imaging object (a pathological specimen, i.e., tissue) is imaged at various magnifications, thereby acquiring, for example, several to several tens of pathological images with different magnifications, and these numerous pathological images are registered in the image DB. Since digitized pathological specimen data can be used, there is an advantage such as it being easy to improve the observation accuracy of a pathologist. A group of pathological images in a virtual slide are substantially identical because they merely represent the same pathological specimen at different magnifications. This concept is the same for any medical image where the same imaging object is imaged at different magnifications. The criterion of substantial identity can also be adopted for region images generated from these substantially identical medical images.

Furthermore, for example, when serial sections are generated by continuously slicing tissue, a certain pathological section and a pathological section immediately above or below it may have almost the same structure. In that case, the pathological images of those respective pathological sections are substantially identical. A similar case is assumed for all medical images other than pathological images. For example, when a three-dimensional structure is sliced, it is assumed that the planar images of adjacent layers are substantially identical. In these cases as well, the plurality of medical images are substantially identical. The criterion of substantial identity can also be adopted for region images generated from these substantially identical medical images.

The embodiments can adopt at least the "criterion of substantial identity of images" described in each of the above examples. For example, in the embodiments, within the image DB of the storage unit 20, the image IDs of a group of medical images that are desired to be treated as substantially identical may be linkable to each other by a user's manual operation or a computer's automatic processing, so as to handle them together.

As an example, it is assumed that a first user (e.g., U001) performs an annotation on a third medical image. It is assumed that a second user (e.g., U002) performs an annotation on a fourth medical image. It is assumed that the fourth medical image is one that is a duplicate of the third medical image, or one that is a duplicate of the third medical image and has had image processing applied to it. The third and fourth medical images may be linked within the image DB, and both images may be treated as identical.

In the embodiments, when a plurality of labels (for example, a first and a second label) are associated with each region image of substantially identical medical images, several examples are conceivable for ID management in each DB of the storage unit 20. In some examples, a grid-method annotation may be performed on a plurality of substantially identical medical images, or a grid setting may be made in a designated-frame-method annotation. In this case, the grid for each medical image may be set with the same size and the same coordinates.

As a first example of ID management, a DB in the storage unit 20 may associate label IDs from each of a plurality of annotations with one region image ID. In this case, a group of region image IDs may be commonly adopted across the plurality of medical images. In other words, simply, a region image ID associated with a certain label (a first label) may also be adopted as a region image ID associated with another label (a second label).

However, it is not limited to this, and a second example of ID management may be as follows. There can be cases where one or more fourth medical images (copied medical images) are generated by copying a third medical image (a master medical image). Furthermore, there are also cases where image processing is applied to the fourth medical image. In at least these cases, although the third and fourth medical images are substantially the same image, they can have different image data and be set with different image IDs. Therefore, as a second example of ID management, the image IDs of the third and fourth medical images may be linked within the image DB. In an image group consisting of a master medical image ID and a group of one or more copied medical image IDs, when a label ID is associated with one region image ID, the same label ID may be automatically associated with the medical images of the remaining image IDs by tracing this association.

At least, the association of a medical image ID, a region image ID, and a label ID may be performed directly as in the first example, or indirectly as in the second example. Thereby, a plurality of annotations may be performed on substantially identical medical images, and a plurality of labels may be associable with one region image by a plurality of annotations.

Note that, for example, the first and second medical images Q, R of the second embodiment are different images from each other and do not fall under substantial identity.

### (4-2. Variations in Server Configuration, etc.)

In the example of FIG. 1, the system 1 is illustrated as one information processing device, but the embodiments are not limited to this. For example, the system 1 may be composed of one or more physical servers or the like, may be configured using a virtual server operating on a hypervisor, or may be configured using a cloud server. Furthermore, the processing of a program related to the generation and display of the selection screen 27 can be arbitrarily shared between the system 1 (a server 1) and the user terminal 3. It should be noted that the way of sharing the processing in each of the processing flows of FIG. 4 to FIG. 6, FIG. 8, FIG. 12, and FIG. 13 is an example.

In the description and claims of the present application, "a computer" should be understood to mean "at least one computer" unless a different meaning is explicitly specified. "A computer" may include any one or more of a user terminal and/or a server. When "a computer" executes a certain program, all of its execution processing may be completed by a single computer, or alternatively, a part of the execution processing may be executed by a first computer (e.g., a server) and the rest of the processing may be executed by one or more other computers (e.g., a user terminal or another server). The one or more other computers may be any one or more of a second to an n-th computer (where n is an arbitrary integer of 3 or more). A generation process of the selection screen may be executed by the user terminal. Alternatively, the generation process of the selection screen may be executed by a server, and in this case, the completed selection screen data may be transmitted to the user terminal. For the generation process of the selection screen, a part of the processing may be executed by the user terminal, and the remaining processing may be executed by a server. However, when it is specified that "a computer" should be interpreted with a meaning different from the above interpretation, it is limited to that description.

While various embodiments according to the present invention have been described, these have been presented as examples and are not intended to limit the scope of the invention. The novel embodiments can be implemented in various other forms, and various omissions, substitutions, and changes can be made without departing from the gist of the invention. The embodiments and their modifications are included in the scope and gist of the invention, and are also included in the scope of the invention described in the claims and their equivalents.

### Reference Signs List

1: system (server), 2: communication network, 3: user terminal, 4: diagnostic device, 11: processor, 12: storage device, 13: communication IF (interface), 20: storage unit, 21: display control unit, 22: input unit, 23: generation unit, 23a: data acquisition unit, 23b, 223b: label determination unit, 23c: training data construction unit, 25, 225: discrimination unit, 26: work screen, 27: selection screen, 31: control unit, 32: storage unit, 33: communication unit, 34: display device, 35: speaker, 36: microphone, 37: camera, 38: operation device, La₁, Laₖ: first label (first information of the first embodiment), Lb₁, Lbₖ: second label (second information of the first embodiment), Q: first medical image, R: second medical image, G: image-attached information (first information of the second embodiment), Lcₖ, Ldₖ: inference classification result (second information of the second embodiment), Leₖ, Lfₖ: label, P: mouse pointer, Q₁, Q₂, Q₃, Q₄, Qₖ, QMₖ, RMₖ, QMⱼ, RMₘ: region image, U001, U002: user

## Claims

1. A program for generating training data from a medical image,
wherein the program causes a computer to execute a generation process,
a plurality of pieces of information are associated with a region image, which is an image of at least one region in the medical image, and
the generation process causes the computer, by a manual operation received via an operation input unit or by an automatic process of the computer, to perform a label association with the region image based on the plurality of pieces of information, thereby generating the training data from the medical image.

2. The program according to claim 1, wherein
the plurality of pieces of information include a first information and a second information,
the first information is a first label,
the second information is a second label, and
the program causes the computer to execute a process of acquiring the first label and the second label associated with one of the region images by a first annotation and a second annotation.

3. The program according to claim 1 or claim 2, wherein
each of the plurality of pieces of information is a label, and
the generation process causes the computer, by a manual operation received via the operation input unit or by an automatic process of the computer, to determine a determined label of the one region image from a plurality of labels associated with the one region image, and to associate a training data image generated from the medical image with the determined label, thereby generating the training data.

4. The program according to claim 1, wherein
the plurality of pieces of information include a first information and a second information,
the region image includes a first region image and a second region image, and
the generation process causes the computer to associate a label determined for the first region image based on the first information with the second region image that has been selected based on the second information, thereby generating the training data.

5. The program according to claim 4, wherein
the program causes the computer to execute a process of acquiring an input data including a plurality of the region images,
a plurality of the first region images included in the plurality of the region images are generated from each of a first medical image and a second medical image,
the first and second medical images are associated with the first information,
the first medical image is an image in which the first information satisfies a predetermined criterion,
the second medical image is an image in which the first information does not satisfy the criterion,
the program causes the computer to execute a discrimination process of causing a trained discrimination model to perform a machine inference for classifying a plurality of the second region images included in the plurality of the region images of the input data,
a classification result of the region image by the machine inference is the second information, and
the generation process causes the computer to associate a label indicating that the first information satisfies the criterion with the second region image that is included in the first region image generated from the first medical image and classified into a first classification group as the classification result, and to associate a label indicating that the first information does not satisfy the criterion with the second region image that is included in the first region image generated from the second medical image and classified into the first classification group as the classification result, thereby generating the training data.

6. The program according to claim 4 or claim 5, wherein
the medical image includes a pathology image.

7. The program according to claim 6, wherein
the first information includes at least one of a gene mutation type, a TMB-H (Tumor Mutational Burden-High) type, a result of a biochemical analysis, or a patient's age, sex, medical history, a result of an immunohistochemical analysis, a lifestyle habit, or a prognostic prediction.

8. The program according to any one of claims 1, 2, 4, and 5, wherein
at least one of the plurality of pieces of information comprises an index value or is an index value, and
the index value represents a certainty of a content of the information.

9. The program according to claim 8, citing claim 2, wherein
a label associated with the region image comprises an index value or is an index value, and
the index value represents how certain a classification result of the label is.

10. The program according to claim 8, citing claim 5, wherein
the trained discrimination model outputs, by the machine inference, a probability value representing a certainty of a classification result of the region image, and
the probability value is used as the index value.

11. A generation method for generating training data from a medical image,
the method causing a computer to execute a generation step,
a plurality of pieces of information are associated with a region image, which is an image of at least one region in the medical image, and
the generation step causes the computer, by a manual operation received via an operation input unit or by an automatic process of the computer, to perform a label association with the region image based on the plurality of pieces of information, thereby generating the training data from the medical image.

12. A system for generating training data from a medical image,
comprising a generation unit,
a plurality of pieces of information are associated with a region image, which is an image of at least one region in the medical image, and
the generation unit, by a manual operation received via an operation input unit or by an automatic process of a computer, performs a label association with the region image based on the plurality of pieces of information, thereby generating the training data from the medical image.
